Europäisches Patentamt

(19) European Patent Office

Office européen des brevets

(11) EP 0 961 111 A2

(12) EUROPEAN PATENT APPLICATION

(43) Date of publication:
01.12.1999 Bulletin 1999/48

(51) Int. Cl.$^6$: **G01N 7/00**, G01N 1/20

(21) Application number: 99109902.9

(22) Date of filing: 20.05.1999

(84) Designated Contracting States:
**AT BE CH CY DE DK ES FI FR GB GR IE IT LI LU MC NL PT SE**
Designated Extension States:
**AL LT LV MK RO SI**

(30) Priority: 29.05.1998 DK 71898

(71) Applicant: **Wolfking Danmark A/S**
**4200 Slagelse (DK)**

(72) Inventor: **Hansen, Tommy Korgard**
**4291 Ruds-Vedby (DK)**

(74) Representative:
**Roerboel, Leif et al**
**Budde, Schou & Ostenfeld A/S,**
**Vestergade 31**
**1456 Copenhagen K (DK)**

(54) **Method and apparatus for automatic in-line measuring the content of compressible gases in a stream of plastic material**

(57) It is the object of the present invention to provide a method with which it is possible to perform an automatic in-line measurement of the content of compressible gases in a flow of plastic material by measuring corresponding values of pressure and volume of a sample taken out from the flow of plastic material, and an apparatus for use when carrying out the method.

Fig. 1.

EP 0 961 111 A2

**Description**

The present invention relates to a method and an apparatus for automatic in-line measuring the content of compressible gases in a flow of plastic material, particularly for measuring the content of air in a meat product, such as sausage meat.

Within the production of sausages there is a wish to measure the content of air in the comminuted sausage meat, as it is desired to reduce the content of air to a suitable low level by means of various evacuation methods.

DK 149,459-B discloses a system related to the present invention. The publication describes - in contrast to the present invention - a system for determining the specific gravity, for providing a measurement for fat content etc. by weighing a material sample and a volume measurement compensating for the content of compressible gases in the sample, in particular air. The compensation is made by using an adjusted volume expressed by $V_{kon} = V - \Delta V \cdot P_2/(P_2-P_1)$ in which V is the volume of the sample at the pressure $P_1$ and $\Delta V$ is the reduction in volume when increasing the pressure from $P_1$ to $P_2$. Even though the above publication indicates the possibility of automating the measuring procedure nothing is indicated as to how this might be possible, and a manual removal of material samples and a manual handling for weighing and measuring of volume is described only.

It is the object of the present invention to provide a method with which it is possible to perform an automatic in-line measurement of the content of compressible gases in a flow of plastic material by measuring corresponding values of pressure and volume of a sample taken out from the flow of plastic material, and an apparatus for use when carrying out the method.

According to the invention this object is achieved by a method as set forth in claim 1 and an apparatus as set forth in claim 5. Especially preferred embodiments of the invention appear from the dependent claims.

In the following the invention is explained in more detail with reference to the drawing showing a preferred embodiment of an apparatus according to the invention for carrying out the method according to the invention.

In the drawing

Fig. 1 shows the apparatus with emptied sample chamber and a closed closure member, and
Fig. 2 shows the apparatus according to Fig. 1 with an open closure member and a filled sample chamber.

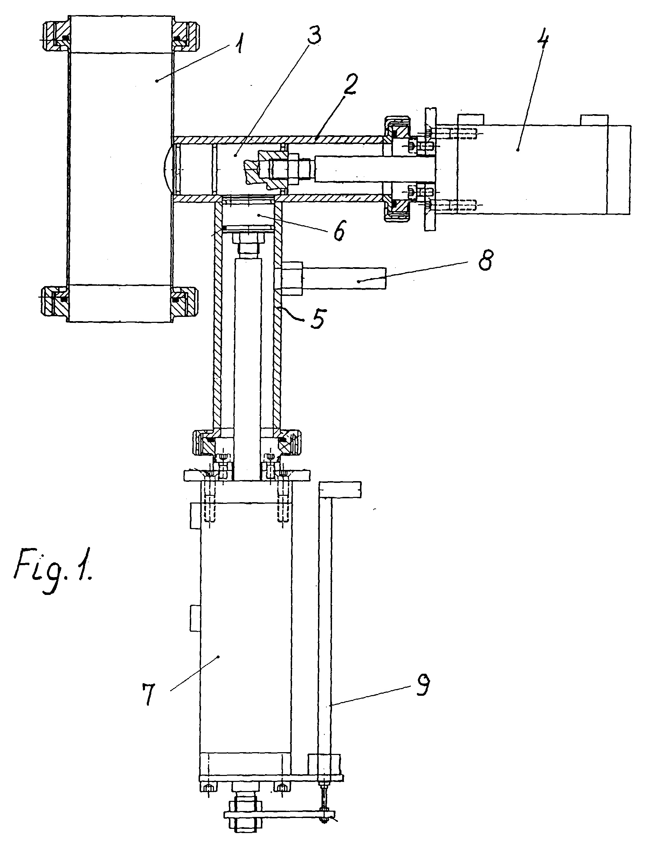

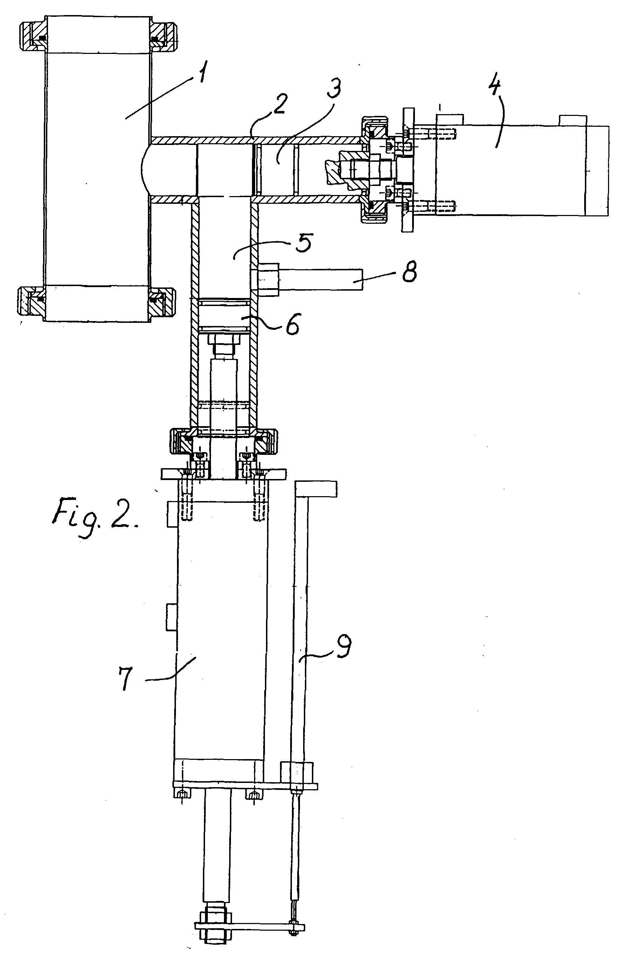

The apparatus shown in the drawing comprises a tube 1 in which the plastic material is flowing of which it is desired to measure the content of compressible gasses. The tube 1 is connected to a cylinder 2 which connects the tube 1 to the cylindrical measuring chamber 5. In the cylinder 2 a piston 3 is positioned working as a closure member for closing the connection between the tube 1 and the measuring chamber 5. The piston 3 may by a drive member 4 be moved between the two positions as shown in Fig. 1 and Fig. 2, respectively. In the measuring chamber 5 a piston 6 is positioned, partly working as a compacting member in connection with the measuring procedure itself and partly working to transfer a material sample to the measuring chamber 5 and to deliver the material sample back to the tube 1 when the measuring procedure has been completed. It is possible to move the piston 6 forwards and backwards in the cylinder of the measuring chamber 5 by means of a drive member 7. The position of the piston 6 in the measuring chamber 5 is detected by means of a position gauge 9 connected with the piston 6 by means of the axle of the drive member 7. For measuring the pressure in the measuring chamber 5 the apparatus shown in the drawing is provided with a pressure transducer 8 placed sideways at the measuring chamber 5.

When starting a measuring cycle the closure piston 3 and the compacting piston 6 are in the positions shown in Fig. 1. The measuring cycle is started by moving the closure piston 3 to the position shown in Fig. 2, thus providing an open connection between the tube 1 and the measuring chamber 5 through the cylinder 2. Then the compacting piston 6 is withdrawn to the position shown in Fig. 2 causing the material to be sucked into the measuring chamber 5. If desired, a certain volume of the material may be transferred to the measuring chamber 5 by stopping the compacting piston 6 in a certain position, which e.g. may be detected by means of a detector placed on the drive member 7, or by means of the position gauge 9. The connection between the tube 1 and the measuring chamber 5 is then closed by moving the closure piston 3 to the position shown in Fig. 1. After this, the desired measurements of corresponding values of pressure, as detected by means of the pressure transducer 8, and volume as detected by means of the position gauge 9, are taken. It is preferred, in the first place, to measure the volume at a pressure approximately equal to atmospheric pressure and subsequently to measure the volume at an increased pressure which preferably is at least 16 times the atmospheric pressure. When the measuring procedure has been completed the pressure in the sample chamber 5 is reduced, preferably to atmospheric pressure, whereafter the closure piston 3 is moved to the position shown in Fig. 2 to permit the material sample to be moved back to the tube 1, the compacting piston being moved to the position shown in Fig. 1, and the closure piston subsequently also being moved to the position shown in Fig. 1. Hereafter, the apparatus is ready for a new measuring cycle.

The measuring procedure described above may by way of example be controlled by means of a programmable logic controller (PLC) which is programmed to control the drive members 4 and 7 for the closure piston and the compacting piston, respectively, and to receive measuring signals from the pressure transducer 8, the position gauge 9, limit switches, if any, for the drive members 4 and 7 and, if it is considered necessary, measuring signals for temperature etc. The programmable logic controller may be programmed to calculate the percentage by volume content of compressible gases by means of the expression $(V_1 - V_2) \cdot P_2 \cdot 100\% : V_1 \cdot (P_2 - P_1)$ where $P_1$, $V_1$ and $P_2$, $V_2$ are corresponding values of pressure and volume for the sample in the measuring chamber 5. The measured volume V may be calculated immediately from the position of the compacting piston 6, as measured by the position gauge 9, since the conversion from the linear measurement from the position gauge 9 to volume is made by multiplication by a constant, which is equal to the sectional area of the measuring chamber 5.

Though the invention in this case is described in connection with a specific embodiment thereof, the invention may be varied within the limits of the subsequent claims, e.g. the closure piston 3 may be replaced by another closure member, e.g. by a plate valve placed in the connection between the measuring chamber 5 and the tube 1, in which case the measuring chamber e.g. may be positioned transversely to the tube 1 so that the compacting piston 6 may be moved transversely to the plate valve to deliver the whole of the material sample to the tube 1 and subsequently take out a new material sample.

Similarly, the measurement of the pressure could be supposed to be performed in another way than by means of a pressure transducer 8, the pressure in the measuring chamber 5 being proportional to the force by which the drive member 7 is influencing the compacting piston 6. When using a known drive member 7, e.g. a pneumatic cylinder, this force may be calculated immediately from operating parameters for the drive member and the known characteristic of the drive member, thus for a pneumatic cylinder the supplied pressure and the area of the piston in the pneumatic cylinder

As drive members 4, 7 it is possible - besides the above pneumatic cylinders - also to use electrically driven drive members or hydraulically driven drive members etc.

## Claims

1. Method for measuring of a parameter for a flow of plastic material by measuring corresponding values of pressure (P) and volume (V) for a sample removed from the flow of plastic material, **characterised** in that the measured parameter is the content of compressible gases and that the measurement is performed in an automated way and in-line, the method comprising the following steps:

   a) Automatic transfer through a connection (2) between the material flow (1) and a sample chamber (5) of a material sample from the material flow to the sample chamber (5), whereafter the connection (2) is closed,
   b) measuring the volume $V_1$ at a first pressure $P_1$ which is preferably equal to the atmospheric pressure (100 kPa),
   c) measuring the volume $V_2$ at an increased second pressure $P_2$ which is preferably at least 16 times the atmospheric pressure (1.6 Mpa),
   d) calculation of the content of compressible gases in the material sample at the first pressure $P_1$, e.g. expressed as a percentage by volume by using the formula:

   $$\text{The percentage by volume content of compressible gases at the pressure } P_1 = \frac{V_1 - V_2}{V_1} \cdot \frac{P_2}{P_2 - P_1} \cdot 100\%$$

   e) opening the connection (2) between the sample chamber (5) and the material flow (1), possibly after reducing the pressure in the sample chamber (5) to $P_1$ and automatic moving the material sample from the sample chamber (5) back to the material flow (1).

2. Method according to claim 1, characterised by the steps a) to e) being repeated at chosen time intervals.

3. Method according to claim 1 or 2, **characterised** in that the plastic material consists of a meat product, preferably a sausage meat product.

4. Method according to any of the previous claims, **characterised** by using a progammable logic controller (PLC) for controlling the automatic transfer of material samples, for measuring, calculating and returning of the material samples, and possibly displaying, printing out, collection, transmission etc. of the results of the measurements.

5. Apparatus for use when carrying out the method according to any of the claims 1 - 4 comprising a measuring chamber (5) having a compacting member (6) placed therein, and means (9) for detecting the position of the compacting member (6) in the measuring chamber (5) in order to provide a measurement of the volume of the measuring chamber, and means (8) to detect the pressure in the measuring chamber (5), **characterised** by further comprising a connection between the measuring chamber (5) and the flowing plastic material (1) and a closure member (3) for closing the measuring chamber (5) from the flow of plastic material (1), the compacting member (6) being constructed to be moved from a position in which an emptying of the measuring chamber (5) is ensured, and a position, in which a filling-up of the measuring chamber (5) with a material sample taken out from the flow of plastic material (1) is ensured, in such a way that the measuring chamber (5) with the taken out sample, after closing the closure member (3) may be subjected to various pressures by means of the compacting member (6), the corresponding volumes being measured by the described detection (9) of the compacting members position in the measuring chamber (5).

6. Apparatus according to claim 5, **characterised** in that the measuring chamber (5) is constructed as a cylinder, and the compacting member (6) is constructed as a piston reciprocably movable in the cylinder of the measuring chamber.

7. Apparatus according to claim 5 or 6, **characterised** by the closure member (3) being constructed as a plate valve closing the cylinder of the measuring chamber (5) from the material flow (1), as the connection (2) to the material flow (1) is constituted by a continuation of the cylinder (5) of the measuring chamber , said cylinder being connected to the material flow (1).

8. Apparatus according to claim 7, **characterised** by the compacting member (6) being constructed to move past the plate valve to the emptying of the measuring chamber (5), and the connection (2) to the material flow (1), said emptying being free of residual material.

9. Apparatus according to claim 5 or 6, **characterised** by the closure member (3) being constructed as a piston being placed in a reciprocably movable manner in a cylinder (2) working as a connection (2) to the material flow (1), and that the measuring chamber cylinder (5) is placed transversely to the connecting cylinder (2), the closure piston (3) extending in such a manner in the connecting cylinder (2) that it closes the cylinder of the measuring chamber (5) from the material flow (1) in the closing position.

10. Apparatus according to any of the claims 5 - 9, **characterised** in that the pressure in the measuring chamber is measured by measuring the force with which the compacting member (6) is influenced.

11. Apparatus according to any of the claims 5-9, characterised in that the pressure in the measuring chamber is measured by means of a pressure transducer (8) connected to the measuring chamber (5).

12. Apparatus according any of the claims 5 - 11, **characterised** by comprising a linear motion transducer (9) connected to the compacting member (6) for detecting of the latter's position in the measuring chamber (5).

13. Apparatus according to any of the claims 5 - 12, **characterised** by comprising a programmable logic controller (PLC) for controlling drive units (4, 7) for the movable closure members and compacting members (3, 6) and for collecting measuring results for pressure and volume and for calculating the desired percentage by volume and for possible registering, printing out, transmission, display etc. of the resulting percentage by volume.

Fig.1.

Fig. 2.